# EUROPÄISCHE PATENTSCHRIFT

(11) **EP 2 653 034 B1**
(45) Veröffentlichungstag und Bekanntmachung des Hinweises auf die Patenterteilung: **23.08.2017**
(21) Anmeldenummer: 13001919.3
(22) Anmeldetag: 12.04.2013
(51) Int. Cl.: A01P 17/00, A01N 65/00, A01N 65/26, A01N 37/02, A01N 25/02

(54) **SPOT-ON PRÄPARAT MIT PARASITEN-REPELLIERENDER WIRKUNG ZUR TOPISCHEN ANWENDUNG AUF TIEREN**
SPOT-ON PREPARATION WITH PARASITE-REPELLING ACTION FOR TOPICAL APPLICATION TO ANIMALS
PRÉPARATION RÉPULSIVE CONTRE LES PARASITES DESTINÉE À UN TRAITEMENT CUTANÉ POUR APPLICATION TOPIQUE SUR DES ANIMAUX

(30) Priorität: 16.04.2012 DE 202012004045 U
(43) Veröffentlichungstag der Anmeldung: 23.10.2013
(73) Patentinhaber: Solnova AG, 8702 Zollikon (CH)
(72) Erfinder: Meyer, Werner, CH-8702 Zollikon (CH)
(74) Vertreter: Sander, Rolf

(56) Entgegenhaltungen:
- WO-A-2011/103694
- WO-A1-2010/108680

## Beschreibung

### Gebiet der Erfindung

Die Erfindung betrifft eine repellierende Zusammensetzung zur topischen Anwendung auf Tieren mit mindestens einem pflanzlichen Repellent als Wirkstoff.

### Hintergrund

Ektoparasiten wie Zecken, Flöhe, Läuse, Haarlinge und Milben werden auf Tieren wie Hunde, Katzen, Pferde, Nager aber auch Tauben und Schlangen häufig gefunden. Ektoparasiten ernähren sich von ihrem Wirtstier und stellen eine ständige Irritationsquelle für das Tier dar. Darüber hinaus haben sie als Überträger von Krankheitserregern Bedeutung erlangt, Zecken z.B. als Überträger von Borreliose und Hirnhautentzündung. Es ist daher wünschenswert, den Kontakt mit solchen Parasiten zu minimieren.

Es gibt bekannte Zusammensetzungen und Verfahren zur Kontrolle und Bekämpfung von Ektoparasiten. Von diesen Produkten sind einige systemische Produkte, d.h. solche, deren Wirkstoffe in die Blutbahn der Tiere eindringen und die insektizide Wirkung entfalten, sobald der Parasit Blut saugt. Systemische Insektizide sind trotz ihrer ausgezeichneten Wirksamkeit weniger wünschenswert, wenn andere, nahezu gleichwertige, äusserlich anwendbare Alternativen bestehen. Während sich systemische Insektizide gegen Flöhe bewährt haben, zeigen sie wenig oder gar keine Wirksamkeit bei der Kontrolle des Zeckenbefalls. Systemisch wirkende Mittel müssen mit Hilfe von Injektionen oder oral verabreicht werden, beides Applikationsformen, die bei den zu behandelnden Tieren und deshalb auch bei den Tierbesitzern wenig beliebt sind.

Auch bereits bekannt sind topisch anwendbare Mittel, z.B. solche, die im Aufsprühverfahren, als Halsband oder als Spot-on zur Anwendung kommen. Solche Produkte sind beispielsweise beschrieben in DE 101 17 676 A1, DE 35 31 920 A1, DE 35 29 693 A1, GB-A-2088212, EP 0 518 989 B1 und DE 691 26 776 T2.

In diesen Schriften werden als Trägerstoffe bzw. Verdünnungsmittel und weitere Hilfsstoffe, wie Emulgatoren, Haftvermittler, Spreitmittel und Tenside, beispielsweise N-Methylpyrrolidon Xylol, Toluol, Cyclohexanon und Alkylglycolether genannt.

Einige dieser Zusammensetzungen können, bedingt durch die enthaltenen Wirkstoffe und/oder Trägerstoffe und/oder Hilfsstoffe, bei ihrer Anwendung, z.B. auf Hunden, an der Anwendungsstelle zu einer vorübergehenden Überempfindlichkeit der Haut, verstärktem Juckreiz, Haarausfall, Rötung und/oder Blasenbildung führen. Diese unerwünschten Effekte sind beispielsweise für den Wirkstoff Permethrin bekannt und abhängig von der Konzentration und Verweildauer des Wirkstoffes auf der Haut.

Alkylglycolether haben hohen Siedepunkt und eine sehr gute Wasserlöslichkeit, sowie gute Mischbarkeit mit organischen Lösungsmitteln. Ihre Eignung als penetrationsfördernde Hilfsmittel ist umstritten, da gelegentlich auch eine Penetrationsreduzierung beobachtet wurde. Für Spot-on-Produkte kann die gute Wasserlöslichkeit des Alkylglykolethers ein Nachteil sein, da bei nicht ausreichend schneller und vollständiger Penetration der Zusammensetzung in die Haut des zu behandelnden Tieres diese beim nächsten Kontakt mit Wasser abgewaschen wird. Dadurch wird die angestrebte Wirkungsdauer gegebenenfalls stark unterschritten.

WO 2005/002339 versucht die oben aufgeführten Nachteile durch eine parasitizide und repellierende Zusammensetzung zu beheben, die zur topischen Anwendung auf domestizierten Tieren bestimmt ist und die eine sehr hohe Konzentration von 50-90 Gew.-% Permethrin oder eines Stereoisomers von Permethrin enthält, sowie Isopropylmyristat in einer zum Erreichen von 100 Gew.-% erforderlichen Menge.

Nachteil dieser Zusammensetzung ist die extrem hohe Permethrin-Konzentration, zumal Permethrin nicht ganz harmlos ist. Zwar hat Permethrin für Warmblüter nur eine geringe akute Toxizität und wird im Körper durch Hydrolyse rasch abgebaut, die US-Umweltbehörde hat Permethrin aber als möglicherweise krebserregend eingestuft. Ferner kann der Kontakt mit dem Stoff bei Menschen zu Juckreiz, Haarausfall und Allergien führen. Für Katzen und Fische ist Permethrin giftig.

Isopropylmyristat, auch bekannt als Myristinsäureisopropylester, Myristinsäure-2-propylester, Muskatbutterderivat oder - abgekürzt - als IPM, ist ein Ester der Myristinsäure (C₁₄-Monocarbonsäure). Myristinsäure kommt beispielsweise vor in Butter, Palmkernöl und Kokosfett. Isopropylmyristat ist eine Ölkomponente, die in der Kosmetik als Spreit- und Rückfettungsmittel sowie als Lösungsvermittler eingesetzt wird. Da es ein Spreitmittel ist, sorgt es für eine gute Verteilung eines Öles oder Fettes auf der Haut. Es ist hautverträglich und dringt leicht in die Haut ein. Verwendung findet es u.a. in Hautcremes, Insektenschutzöl, Sonnenschutzöl, Lippenstiften u. Ä.

Kommerziell erhältliche Spot-on-Produkte auf natürlicher Basis enthalten beispielsweise als aktive Bestandteile 3.0% Pfefferminzöl, 4.5% Zimtöl, 4.5% Zitronengrasöl, 5.0% Nelkenöl, 5.0% Thymianöl, 5.0% 2-Phenethylpropionat, Vanille, und 73.0% Isopropyl Myristat.

Margosa-Extrakt oder Margosa-Öl wird aus dem Samen der reifen Blüten des Neembaumes gewonnen und ist bekannt als Biozid. Es wird bereits - zusammen mit Citronella und Nelkenblütenöl - in hoher Konzentration von 16 Gew.-% in Spot-on-Produkten (bogacare®)angeboten, über weitere Inhaltstoffe ist aber nichts bekannt. Ebenfalls bekannt ist eine Zusammensetzung enthaltend 3 Gew.-% Margosa-Extrakt, 9.8 Gew.-% Ethylbutylacetoaminopropionat und 86.95 Gew.-% des gut wasserlöslichen Ethyldiglykol (Bob Martin® Neem Spot-on).

In EP 2 410 861 A1 und WO 2011/103694 A1 werden Spot-on Produkte beschrieben, die neben Neem-Extrakt bzw. Margosa hauptsächlich Isopropylmyristat enthalten. Solche Produkte, insbesondere die Kombination von Margosa-Extrakt mit Decansäure zeigte sehr gute Wirksamkeit gegen Flöhe und Zecken, sie sind aber hinsichtlich Geruch und Fettigkeit nicht befriedigend. Insbesondere bei langhaarigen Tieren, können bei unvorsichtigem Auftrag fettige Stellen entstehen.

Ziel der vorliegenden Erfindung war es deshalb, eine Ektoparasiten repellierende Zusammensetzung mit möglichst geringer Beeinträchtigung des behandelten Säugetiers, geringer Umwelttoxizität und langer Wirksamkeit bereitzustellen, das gleichzeitig wenig oder nicht riecht und reduzierte Fettigkeit/Öligkeit aufweist. Insbesondere eine repellierende Zusammensetzung mit natürlichen Wirkstoffen.

### Darstellung der Erfindung

Da bei Repellent-Produkten die Parasiten durch den von der Haut verdampfenden Wirkstoff über den Geruchssinn, bzw. bei Kontakt des Parasiten mit der Haut des Wirtstiers repelliert werden, war es höchst erstaunlich, dass durch Änderung des Spreitmittels der Geruch und die Öligkeit bereits direkt nach der Anwendung verbessert werden kann, und dies bei zumindest gleichbleibender Wirksamkeit.

Erfindungsgemässe repellierende Zusammensetzung zur topischen Anwendung auf Säugetieren zeichnen sich aus durch 3 bis 12 Gew.-% einer Wirkstoffkomponente, 0 bis 5 Gew.-% Hilfsstoffen und dem Rest Isopropyllaurat und Isopropylmyristat in einem Verhältnis von Isopropyllaurat zu Isopropylmyristat von 70:30 bis 100:0, wobei die Wirkstoffkomponente aus einem oder mehreren natürlichen Repellents besteht, von denen mindestens 90 Gew.-% Margosa-Extrakt oder einer Mischung aus Margosa-Extrakt und Decansäure sind.

Isopropyllaurat ist ein erfindungsgemäß bevorzugter C6-C12-Fettsäureester.

Unter geringer Wirkstoffkonzentration wird eine Konzentration von maximal ca. 12 Gew.-% verstanden, insbesondere eine Konzentration von 3 bis 12 Gew.-%, vorzugsweise eine Konzentration von 4 bis 8 Gew.-%, wie eine Konzentration von 5 bis 8 Gew.-% oder 5 bis 7 Gew.-%.

Erfindungsgemäß geeignete Wirkstoffe/Repellents sind Margosa-Extrakt/Margosa-Öl (in der Folge nur als Margosa-Extrakt bezeichnet) oder eine Mischung aus Margosa-Extrakt und Decansäure. Diese machen erfindungsgemäss mindestens 90 Gew.-% der gesamten Wirkstoffmenge aus, vorzugsweise ca. 100 Gew.-%. Als ein Trägerstoff oder Spreitmittel wird im Rahmen der Erfindung in Wasser schwer- bis unlösliches Spreitmittel, in Gestalt von Isopropyllaurat und Isopropylmyristat eingesetzt.

Überraschenderweise wurde festgestellt, dass C6-C12-Fettsäureester, insbesondere Isopropyllaurat, obschon sie neben der Funktion als Spreitmittel zu verstärkter Hautpenetration führen, die Repellent Wirkung nicht beeinträchtigen, aber den Geruch und die Öligkeit, welche dem Margosa-Extrakt zugeschrieben werden, stark reduzieren. Es wird angenommen (ohne dass der Erfinder in irgendeiner Weise durch seine Theorie gebunden sein will), dass die überraschend guten Eigenschaften der Zusammensetzung auf einem ausgewogenen Verhältnis zwischen der Spreitung, bzw. der Verteilung auf einer möglichst grossen Hautfläche, der geringen Wasserlöslichkeit und der Penetration beruhen, wobei angenommen wird, dass ein Transport von Wirkstoff in die Haut nur in die äussersten Hautschichten, z.B. nur in die Oberhaut (Epidermis), erfolgt, so dass infolge des Konzentrationsgefälles zwischen der Hautoberfläche und der Epidermis mit der Zeit wieder Repellent-Mengen aus der Haut an die Hautoberfläche gelangen und dort ihre Wirkung entfalten.

Durch die hydrophoben Eigenschaften der Repellentien und der Spreitmittel in Kombination mit allfälliger Penetration eines Teils der Zusammensetzung in die Haut, wird ein Abwaschen des Wirkstoffs bei Wasserkontakt, z.B. im Regen, verhindert, so dass die Langzeitwirkung gegeben ist.

Unter Spreitmitteln werden allgemein ölige Flüssigkeiten verstanden, die sich auf der Haut besonders gut verteilen (siehe z.B. R. Keymer, Pharm. Ind. 32, 577,(1970)). Beispiele für Spreitmittel sind:
- Silikonöle verschiedener Viskosität
- Fettsäureester, wie Ethylstearat, Di-n-butyl-adipat, Laurinsäurehexylester, Dipropylenglykolpelargonat, Ester einer verzweigten Fettsäure mittlerer Kettenlänge mit gesättigten Fettalkoholen C16-C18, Isopropylmyristat, Isopropylpalmitat, Capryl/Caprinsäureester von gesättigten Fettalkoholen der Kettenlänge C12-C18, Isopropylstearat, Ölsäureoleylester, Ölsäuredecylester, Ethyloleat, Milchsäureethylester, Cetearylisononanoate, Decylolea, Ethylhexylpalmitat, wachsartige Fettsäureester wie künstliches Entenbürzeldrüsenfett, Dibutylphthalat, Adipinsäurediisopropylester, letzterem verwandte Estergemische
- Triglyceride, wie Capryl/Caprinsäuretriglycerid, Triglyceridgemische mit natürlichen Fettsäuren, Partialglyceridgemische gesättigter oder ungesättigter evtl. auch hydroxylgruppenhaltige Fettsäuren, Monoglyceride der C8/C10-Fettsäuren
- Fettalkohole, wie Isotridecylalkohol, 2-Octyldodecanol, Cetylstearyl-Alkohol, Oleylalkohol und
- Fettsäuren, wie z.B. Ölsäure.
- Ether, wie Dicaprylylether, natürliche Öle wie Sonnenblumenöl, Avocadoöl.

Bevorzugte Spreitmittel sind in Wasser schwer- bis unlösliche Spreitmittel.

Erfindungsgemäß werden Isopropylmyristat und Isopropylpalmitat in dem in Anspruch 1 genannten Verhältnis eingesetzt.

Neben dem Wirkstoff und dem Spreitmittel bzw. der Spreitmittelzusammensetzung können die repellierenden Zusammensetzungen weitere Träger- und/oder Hilfsstoffe enthalten, wie die homogene Wirkstoffverteilung (in Lösungs- oder Emulsionsform) fördernde Mittel, Haftvermittler, Stabilisatoren, Filmbildner, Konditionierer, Parfüms, Fellglanzmittel und Farbagenzien. Solche Stoffe sind für die Verwendung in Kosmetika bekannt. Weitere Träger- und/oder Hilfsmittel kommen üblicherweise in Mengen von maximal 10 Gew.-% vor, vorzugsweise maximal 5 Gew.-%, insbesondere maximal 2 Gew.-% und speziell bevorzugt maximal ca. 1 Gew.-%. Bevorzugte Zusammensetzungen bestehen aus dem Wirkstoff Margosa-Extrakt oder einer Mischung aus Margosa-Extrakt und Decansäure, und dem Isopropylmyristat, oder sie enthalten zusätzlich maximal 2 Gew.-% Hilfsstoffe, z.B Parfüms, wobei für Parfüms dank der erfindungsgemässen Zusammensetzung normalerweise eine Menge von weit weniger als 1 Gew.-% ausreicht um einen unangenehmen Restgeruch der Wirkstoffkomponente zu überdecken.

### Kurze Beschreibung der Zeichnungen

Die Resultate der unten beschriebenen Experimente sind in den Figuren dargestellt. Dabei zeigen:
Figur 1 die Durchschnittswerte des Flohbefalls in Abhängigkeit der Zeit ab Behandlung und
Figur 2 die Durchschnittswerte des Zeckenbefalls in Abhängigkeit der Zeit ab Behandlung

### Weg(e) zur Ausführung der Erfindung

Im Gegensatz zu Parasiten abtötenden Produkten, die meist Wirkstoffe wie Fipronil, Permethrin etc. enthalten, die als Arzneimittel zugelassen werden müssen, enthalten viele Repellent-Produkte nätürliche Wirkstoffe und werden als Biozide eingestuft.

Bekannte Repellent-Wirktoffe sind beispielsweise etherische Öle, z.B. Margosa-Extrakt (aus der Neem-Pflanze gewonnen und deshalb auch als Neem-Extrakt oder Neemöl bekannt) und Decansäure (aus Palmöl), üblicherweise mit verschiedenen Ölen (Triglyceriden) als Trägersubstanz (Excipiens).

Unter der Bezeichnung Margosa-Extrakt, Margosa-Öl werden unterschiedlich gewonnene Produkte verstanden.

Ein bevorzugter Margosa Extrakt ist ein beispielsweise mit CO₂ unter hohem Druck aus den Samen von Azadirachta indica (Neem-Samen) extrahierter Margosa-Extrakt. Ein solcher Extrakt ist erhältlich von Terra Nostra GmbH, Geisenfeld, Deutschland. Als Hauptbestandteile eines solchen Extraktes werden Fettsäuren, beispielsweise Ölsäure, Stearinsäure, Linolsäure und Palmitinsäure genannt, die als freie Fettsäuren und/oder Triglyceride vorliegen können. Weitere übliche Inhaltsstoffe umfassen Limonoide, wie z.B. Nimbin, Salannin und Azadirachtin. Ferner sollte ein solcher Extrakt gemäß Mykotoxin-Höchstmengenverordnung (MHmV) frei von Aflatoxinen sein. Ein solches Margosa-Extrakt entspricht der CAS-Nr.: 84696-25-3.

Mit Hinweis auf die gleiche CAS-Nr. (CAS-Nr.: 84696-25-3) wird von Sigma-Aldrich ein wässriger, propylenglykolhaltiger Pflanzenextrakt aus der Neem-Rinde angeboten (siehe http://www.sigmaaldrich.com/catalog/product/fluka/73279?l ang=de&region=CH).

Ebenfalls bevorzugt ist ein kaltgepresstes Margosa-Extrakt bzw. Margosa-Öl, das erhältlich ist von der Firma Plasma Power Private Limited, Chennai, Indien unter der Bezeichnung Plasma Neem® oil. Für dieses Produkt wird die CAS Nr: 8002-65-1 angeführt.

Großer Nachteil der Neem-Produkte ist deren unangenehmer Geruch und deren unangenehme Öligkeit.

Im Rahmen der vorliegenden Erfindung wurde nun festgestellt, dass bei Verwendung von C6-C12 Fettsäureestern als Trägermittel und Spreitmittel - obschon sich auch diese ölig anfühlen, die Öligkeit der Gesamtzusammensetzung nach Anwendung stark vermindern lässt und dies bei gleichzeitig stark reduziertem Geruch und gleichbleibender Wirksamkeit.

Die repellierenden Zusammensetzungen zur topischen Anwendung auf Säugetieren gemäss der vorliegenden Erfindung, bestehen aus 3 bis 12 Gew.-% einer Wirkstoffkomponente, mindestens 80 Gew.-% einer Spreitmittelkomponente und 0 bis 10 Gew.-% Träger- und/oder Hilfsstoffen, wobei die Wirkstoffkomponente aus einem oder mehreren natürlichen Repellents besteht, wovon mindestens 50 Gew.-% Margosa-Extrakt und/oder Decansäure sind, und wobei die Spreitmittelkomponente zu mindestens 50 Gew.-% aus C6-C12 Fettsäureester besteht.

In bevorzugten Zusammensetzungen machen Margosa und/oder Decansäure mindestens 70 Gew.-%, insbesondere mindestens 90 Gew.-% und speziell bevorzugt ca. 100 Gew.-% des repellierenden Wirkstoffs aus.

In einer weiteren bevorzugten Ausführugnsform besteht die Trägerkomponente bzw. das Spreitmittel aus 50-100 Gew.-% C6-C12-Fettsäureester, insbesondere Isopropyllaurat, und mindestens 10 Gew-% des verbleibenden Anteils aus Isopropylmyristat, d.h.
- x Gew.-% C6-C12-Fettsäureester
- 100-x Gew.-% andere Spreitmittel, davon y Gew.-% Isopropylmyristat, entsprechend y(100-x) Gew.-% Isopropylmyristat bezogen auf die gesamte Spreitmittelmenge

In einer bevorzugten Ausführungsform besteht die Zusammensetzung aus 3 bis 12 Gew.-% eines natürlichen Repellents, insbesondere 5 bis 8 Gew.-%, sowie 0 bis 5 Gew.-% Hilfsstoffen, insbesondere 0 bis 1 Gew.-% Hilfsstoffen, insbesondere weit unter 1 Gew.-% Parfum, mindestens 50 Gew.-% Isopropyllaurat und dem Rest Isopropylmyristat.

Insbesondere wurde gefunden, dass eine Zusammensetzung aus 4 Gew.-% Margosa und 4 Gew.-% Decansäure und (i) 92 Gew.-% Isopropyllaurat oder (ii) 92 Gew.-% Isopropyllaurat und Isopropylmyristat im Verhältnis 9:1 und (iii) 6 Gew.-% Margosa und 94 Gew.-% Isopropyllaurat oder (iv) 6 Gew.-% Decansäure und 94 Gew.-% Isopropyllaurat bei guter Wirksamkeit kaum zu unangenehmem Geruch oder öligem Fell führte.

Für Gegenden mit sehr hoher Konzentration an Ektoparasiten oder bei Säugetieren mit hoher Anfälligkeit, kann es sinnvoll und bevorzugt sein eine höher konzentrierte Zusammensetzung zu wählen bzw. anzuwenden, beispielsweise von 8 bis 12 Gew.-%, insbesondere von 9 bis 10 Gew-%. Auch so hoch konzentrierte Zusammensetzungen wiesen in ersten Versuchen an Hunden und Katzen keine inakzeptablen Nebenwirkungen auf.

Die Wirkstoffkomponente kann neben Margosa-Extrakt und/oder Decansäure auch Knoblauchextrakt, Eucalyptus citriodora, Tomatenextrakt, essentielle Öle wie Pfefferminze, Zedernholzextrakt, Zitronellaöl, Extrakte aus Citrus hystrix, Extrakte aus Cymbopogon winterianus, Extrakte aus Ocimum americanum und Mischungen derselben umfassen.

Bevorzugte Mischungen aus Margosa-Extrakt und Decansäure sind Mischungen im Verhältnis 30:70 bis 70:30, insbesondere im Verhältnis 40:60 bis 60:40, speziell bevorzugt ca. 50 : 50.

Solche Zusammensetzungen sind grundsätzlich für die topische Applikation auf allen Säugetieren, einschliesslich Menschen geeignet. Bevorzugt ist die Anwendung als Spot-on-Formulierung. Eine andere Anwendungsart ist in Form eines Halsbands. Solche Halsbänder können aus mit der erfindungsgemässen Zusammensetzung imprägniertem, saugfähigem Material hergestellt werden.

Bevorzugte Haus- und Heimtiere für die Spot-on Anwendung sind Hund und Katze, aber auch Nager, Meerschweinchen, Tauben und Schlangen. Die Anwendung als Halsband eignet sich insbesondere für Hunde und Katzen.

Die erfindungsgemässen Zusammensetzungen lassen sich durch einfaches Zusammenmischen der Inhaltsstoffe herstellen.

Für die Anwendung als Spot-on-Produkt werden die Zusammensetzungen für Hunde und Katzen vorzugsweise in Einheitsmengen von 1 ml bis 2 ml in Ampullen abgefüllt. Solche Ampullen sind für Spot-on Produkte bekannt.

Alternativ können die Zusammensetzungen in Sprühdosen abgefüllt werden, z.B. zur Behandlung grosser Tiere, beispielsweise von Pferden.

C6-C12-Fettsäureester, wie Isopropyllaurat, können auch zur Reduzierung des Geruchs und der Öligkeit von topisch zu applizierenden, Repellent-Wirkstoffe enthaltenden Zusammensetzungen verwenden, die nicht in die bevorzugten Grenzen der oben genannten Zusammensetzungen fallen.

### Beispiele:

### Beispiele 1 bis 4:Untersuchte Rezepturen:

**Tabelle 1:**

| **Inhaltsstoff** | **Rezeptur** | | | |
|---|---|---|---|---|
| | **1** | **2** | **3** | **4** |
| Isopropylmyristat | 92 | 0 | 0 | 0 |
| Isopropylmyristat: Isopropyllaurat 1:9 | 0 | 92 | 0 | 0 |
| Isopropyllaurat | 0 | 0 | 92 | 94 |
| Margosa Extrakt | 4 | 4 | 4 | 6 |
| Decansäure | 4 | 4 | 4 | 0 |

### Wirkung auf Katzen

Als Tiere für die Untersuchung wurden Katzen gewählt, da bei diesen Nebenwirkungen besser in Erscheinung treten als beispielsweise bei Hunden.

### Auswahl der Versuchstiere

Bei den für die Untersuchung gewählten Katzen handelt es sich um Tiere, die aus einem Tierheim und dem Bekanntenkreis des Laborpersonals akquiriert wurden. Um den Stress der Tiere möglichst gering zu halten, blieben diese über die gesamte Versuchsdauer unter Obhut ihrer Halter und wurden nur für die Versuche in das Labor gebracht.

### Anwendung des Produktes

Es wurden jeweils 2 ml des Testproduktes mit einer geeichten Plastikpipette direkt auf die Haut der Katzen in einem Strich vom Hinterkopf bis zur Schwanzwurzel aufgetragen. Um sicherzustellen, dass das Produkt direkt auf die Haut und nicht auf das Fell aufgetragen wird, wurde das Fell der Katzen direkt vor bzw. während dem Auftragen entlang dieses Striches auseinandergekämmt.

### Versuchsanordnung

Die Versuchstiere wurden zu Beginn des Versuches auf Art und Anzahl des Befalles untersucht und nach dessen Dokumentation, wie oben beschrieben mit dem Testprodukt behandelt. Danach wurde alle Tiere wieder in ihre ursprünglichen Bereiche entlassen und in den unten angegebenen Zeitintervallen auf Geruch, Öligkeit und vorhandene Parasiten untersucht bis erneut Befall - naturgemäss von Flöhen und/oder Zecken - vorlag. Die 50 Probanden wurden jeweils in den Monaten Juli bis November 2011 getestet. Die Zeit bis zur vollkommenen Befallsfreiheit wird als Austreibungszeit; die Zeit von der ersten festgestellten Befallsfreiheit bis zum ersten Befall wird als Schutzzeit bezeichnet. Zur Kontrolle wurden gleichzeitig Tiere mit Mittel ohne Wirkstoffe getestet, um den Befallsdruck unter den Testbedingungen zu messen.

Neben den einzelnen Rezepturen wurde auch eine Kontrollgruppe (K1 bis K10) untersucht, die keinerlei Behandlung erfahren hat.

Die Resultate dieser Kontrollgruppe sind in den Tabellen 2 und 3 zusammengestellt.

Die mit der Rezeptur 1 (Stand der Technik) erhaltenen Resultate sind in den Tabellen 4 und 5 wiedergegeben und die Resultate der erfindungsgemässen Rezeptur 2 in den Tabellen 6 und 7, die Resultate der erfindungsgemässen Rezeptur 3 in den Tabellen 8 und 9 und die Resultate der erfindungsgemässen Rezeptur 4 in den Tabellen 10 und 11.

Die Wirksamkeit wurde anhand des Parasitenbefalls bestimmt. Dieser ist in den nachfolgenden Tabellen dargestellt. Darin bedeuten:
t: Zeitpanne nach Auftrag des Produktes
h: Stunden
d: Tage in Tagen
t = 0h: Bestimmung des Befalls unmittelbar vor Auftragen der jeweiligen Rezeptur
F: Flöhe; Z: Zecken

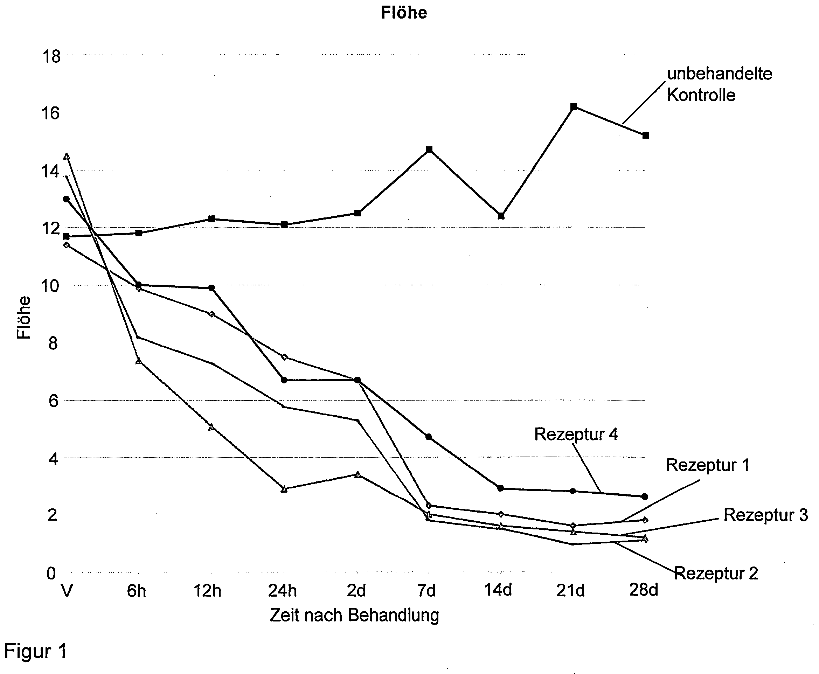

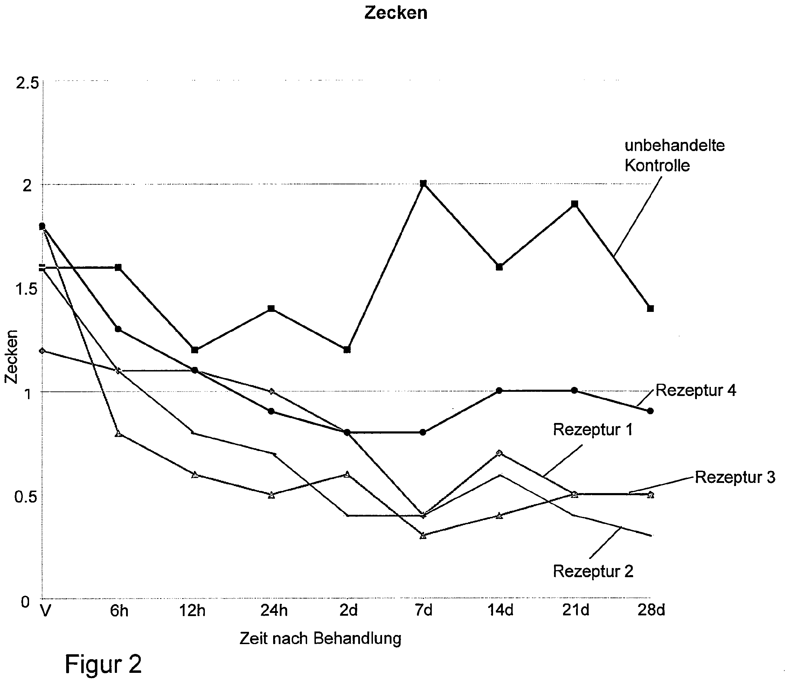

Die letzte Zeile in den Tabellen 3,5,7,9 und 11 gibt die Mittelwerte des Befalls wieder. Diese sind in der Tabelle 12 zusammengefasst und in den Figuren 1 und 2 als Diagramme dargestellt.

**Tabelle 2 - Kontrollgruppe**

| | **6h** | | **12h** | | **24h** | | **2 Tage** | |
|---|---|---|---|---|---|---|---|---|
| | **Geruch** | **fettig** | **Geruch** | **fettig** | **Geruch** | **fettig** | **Geruch** | **fettig** |
| K1 | - - | - - | - - | - - | - - | - - | - - | - - |
| K2 | - - | - - | - - | - - | - - | - - | - - | - - |
| K3 | - - | - - | - - | - - | - - | - - | - - | - - |
| K4 | - - | - - | - - | - - | - - | - - | - - | - - |
| K5 | - - | - - | - - | - - | - - | - - | - - | - - |
| K6 | - - | - - | - - | - - | - - | - - | - - | - - |
| K7 | - - | - - | - - | - - | - - | - - | - - | - - |
| K8 | - - | - - | - - | - - | - - | - - | - - | - - |
| K9 | - - | - - | - - | - - | - - | - - | - - | - - |
| K10 | - - | - - | - - | - - | - - | - - | - - | - - |

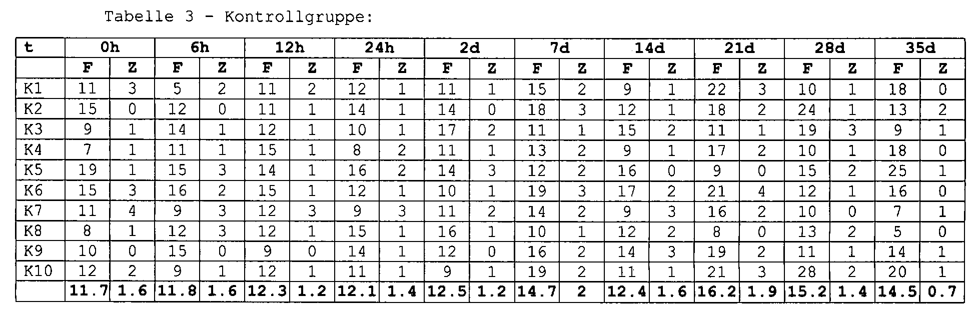

**Tabelle 4 - Rezeptur 1 (Stand der Technik)**

| | **6h** | | **12h** | | **24h** | | **2 Tage** | |
|---|---|---|---|---|---|---|---|---|
| | **Geruch** | **fettig** | **Geruch** | **fettig** | **Geruch** | **fettig** | **Geruch** | **fettig** |
| K11 | + | + | + | + | +- | + | - - | - - |
| K12 | ++ | + | + | + | + | + | - - | - - |
| K13 | ++ | + | ++ | + | +- | + | - - | - - |
| K14 | + | + | + | - | - | - - | - - | - - |
| K15 | ++ | + | ++ | + | + - | - | - - | - - |
| K16 | +- | + | +- | + | + - | + | - - | - - |
| K17 | + | ++ | + | ++ | + - | + | - - | - - |
| K18 | + + | + | + | + | + | + | - - | - - |
| K19 | + | + | + - | + | + - | + | - - | - - |
| K20 | + + | + | + | + | + - | + | - - | - - |

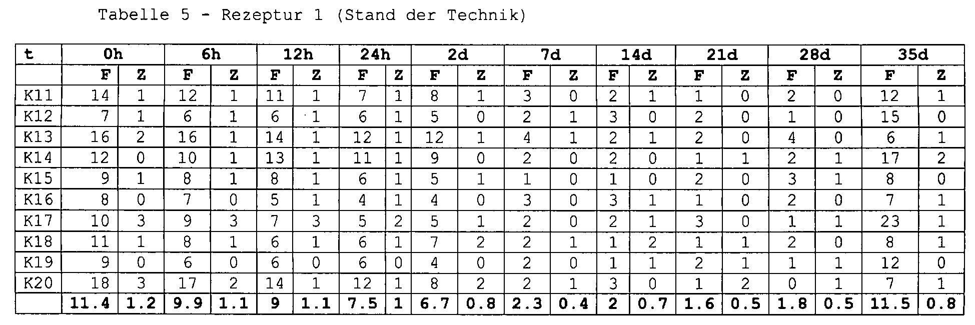

**Tabelle 6 - Rezeptur 2 (erfindungsgemäss)**

| | **6h** | | **12h** | | **24h** | | **2 Tage** | |
|---|---|---|---|---|---|---|---|---|
| | **Geruch** | **fettig** | **Geruch** | **fettig** | **Geruch** | **fettig** | **Geruch** | **fettig** |
| K21 | - - | - - | - - | - - | - - | - - | - - | - - |
| K22 | - - | - - | - - | - - | - - | - - | - - | - - |
| K23 | + - | - - | + - | - - | - - | - - | - - | - - |
| K24 | - - | - - | - - | - - | - - | - - | - - | - - |
| K25 | + | - - | - - | - - | - - | - - | - - | - - |
| K26 | - - | - - | - - | - - | - - | - - | - - | - - |
| K27 | - - | + | - - | + | - - | - - | - - | - - |
| K28 | + - | - - | - - | - - | - - | - - | - - | - - |
| K29 | - - | - - | - - | - - | - - | - - | - - | - - |
| K30 | - - | - - | - - | - - | - - | - - | - - | - - |

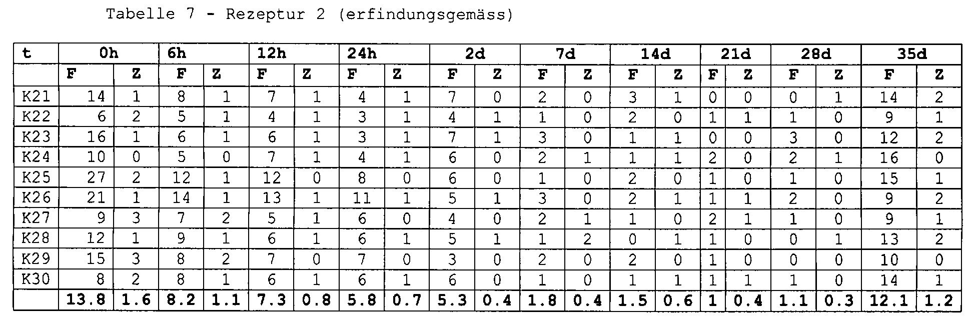

**Tabelle 8 - Rezeptur 3 (erfindungsgemäss)**

| | **6h** | | **12h** | | **24h** | | **2 Tage** | |
|---|---|---|---|---|---|---|---|---|
| | **Geruch** | **fettig** | **Geruch** | **fettig** | **Geruch** | **fettig** | **Geruch** | **fettig** |
| K31 | - - | - - | - - | - - | - - | - - | - - | - - |
| K32 | - - | - - | - - | - - | - - | - - | - - | - - |
| K33 | - - | - - | - - | - - | - - | - - | - - | - - |
| K34 | - - | - - | - - | - - | - - | - - | - - | - - |
| K35 | - - | - - | - - | - - | - - | - - | - - | - - |
| K36 | - - | - - | - - | - - | - - | - - | - - | - - |
| K37 | - - | - - | - - | - - | - - | - - | - - | - - |
| K38 | - - | - - | - - | - - | - - | - - | - - | - - |
| K39 | - - | - - | - - | - - | - - | - - | - - | - - |
| K40 | - - | - - | - - | - - | - - | - - | - - | - - |

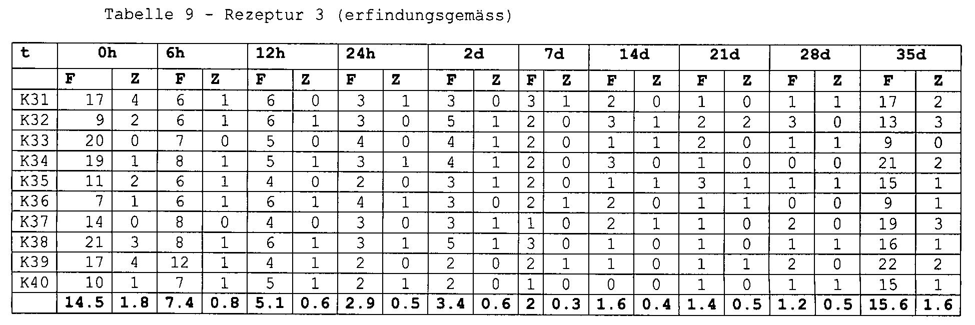

**Tabelle 10 - Rezeptur 4 (erfindungsgemäss)**

| | **6h** | | **12h** | | **24h** | | **2 Tage** | |
|---|---|---|---|---|---|---|---|---|
| | **Geruch** | **fettig** | **Geruch** | **fettig** | **Geruch** | **fettig** | **Geruch** | **fettig** |
| K41 | - - | - - | - - | - - | - - | - - | - - | - - |
| K42 | + | - - | - - | - - | - - | - - | - - | - - |
| K43 | - - | + - | - - | - - | - - | - - | - - | - - |
| K44 | - - | - - | - - | - - | - - | - - | - - | - - |
| K45 | + - | - - | - - | - - | - - | - - | - - | - - |
| K46 | - - | - - | - - | - - | - - | - - | - - | - - |
| K47 | - - | - - | - - | - - | - - | - - | - - | - - |
| K48 | - - | - - | - - | - - | - - | - - | - - | - - |
| K49 | - - | + - | - - | - - | - - | - - | - - | - - |
| K50 | - - | - - | - - | - - | - - | - - | - - | - - |

**Tabelle 11 - Rezeptur 4 (erfindungsgemäss)**

| t | 0h | | 6h | | 12h | | 24h | | 2d | | 7d | | 14d | | 21d | | 28d | | 35d | |
|---|---|---|---|---|---|---|---|---|---|---|---|---|---|---|---|---|---|---|---|---|
| | F | Z | F | Z | F | Z | F | Z | F | Z | F | Z | F | Z | F | T | F | T | F | T |
| K41 | 15 | 2 | 14 | 1 | 12 | 1 | 9 | 1 | 8 | 1 | 5 | 0 | 3 | 1 | 3 | 1 | 2 | 0 | 12 | 1 |
| K42 | 12 | 2 | 11 | 2 | 10 | 1 | 6 | 1 | 5 | 0 | 4 | 1 | 5 | 0 | 5 | 1 | 3 | 2 | 15 | 0 |
| K43 | 16 | 1 | 14 | 1 | 15 | 1 | 10 | 1 | 12 | 1 | 6 | 1 | 4 | 1 | 2 | 1 | 4 | 2 | 6 | 1 |
| K44 | 11 | 1 | 11 | 1 | 12 | 1 | 9 | 1 | 9 | 0 | 6 | 1 | 2 | 2 | 4 | 1 | 3 | 1 | 17 | 2 |
| K45 | 12 | 2 | 7 | 1 | 9 | 1 | 5 | 1 | 5 | 1 | 3 | 0 | 2 | 0 | 2 | 0 | 3 | 1 | 8 | 0 |
| K46 | 10 | 1 | 5 | 1 | 6 | 1 | 3 | 1 | 4 | 0 | 5 | 0 | 3 | 1 | 4 | 1 | 3 | 0 | 7 | 1 |
| K47 | 9 | 2 | 8 | 3 | 8 | 2 | 2 | 1 | 5 | 1 | 7 | 1 | 2 | 1 | 3 | 1 | 2 | 1 | 23 | 1 |
| K48 | 14 | 3 | 5 | 1 | 6 | 1 | 5 | 1 | 7 | 2 | 4 | 1 | 3 | 2 | 2 | 1 | 4 | 0 | 8 | 1 |
| K49 | 10 | 1 | 8 | 0 | 6 | 1 | 6 | 0 | 4 | 0 | 2 | 2 | 2 | 1 | 2 | 1 | 1 | 1 | 12 | 0 |
| K50 | 21 | 3 | 17 | 2 | 15 | 1 | 12 | 1 | 8 | 2 | 5 | 1 | 3 | 0 | 1 | 2 | 1 | 1 | 7 | 1 |
| | **13** | **1.8** | **10** | **1.3** | **9.9** | **1.1** | **6.7** | **0.9** | **6.7** | **0.8** | **4.7** | **0.8** | **2.9** | **0.9** | **2.8** | **1** | **2.6** | **0.9** | **11.5** | **0.8** |

Eine Zusammenstellung der Mittelwerte, welche auch in den Figuren 1 und 2 dargestellt sind, ist der Tabelle 12 zu entnehmen

**Tabelle 12:**

| **Flöhe** | **V** | **6h** | **12h** | **24h** | **2d** | **7d** | **14d** | **21d** | **28d** |
|---|---|---|---|---|---|---|---|---|---|
| unbehandelte Kontrolle | 11.7 | 11.8 | 12 | 12.1 | 12.5 | 15 | 12.4 | 16.2 | 15.2 |
| Rezeptur l:Myristrat | 11.4 | 9.9 | 9 | 7.5 | 6.7 | 2.3 | 2 | 1.6 | 1.8 |
| Rezeptur 2: Myristrat:Laurat 1:9 | 13.8 | 8.2 | 7.3 | 5.8 | 5.3 | 1.8 | 1.5 | 1 | 1.1 |
| Rezeptur 3: Laurat | 14.5 | 7.4 | 5.1 | 2.9 | 3.4 | 2 | 1.6 | 1.4 | 1.2 |
| Rezeptur 4: nur Margosa /Laurat | 13 | 10 | 9.9 | 6.7 | 6.7 | 4.7 | 2.9 | 2.8 | 2.6 |

| **Zecken** | **V** | **6h** | **12h** | **24h** | **2d** | **7d** | **14d** | **21d** | **28d** |
|---|---|---|---|---|---|---|---|---|---|
| unbehandelte Kontrolle | 1.6 | 1.6 | 1.2 | 1.4 | 1.2 | 2 | 1.6 | 1.9 | 1.4 |
| Rezeptur l:Myristrat | 1.2 | 1.1 | 1.1 | 1 | 0.8 | 0.4 | 0.7 | 0.5 | 0.5 |
| Rezeptur 2:Myristrat 10%/Laurat 90% | 1.6 | 1.1 | 0.8 | 0.7 | 0.4 | 0.4 | 0.6 | 0.4 | 0.3 |
| Rezeptur 3: Laurat | 1.8 | 0.8 | 0.6 | 0.5 | 0.6 | 0.3 | 0.4 | 0.5 | 0.5 |
| Rezeptur 4: nur Margosa /Laurat | 1.8 | 1.3 | 1.1 | 0.9 | 0.8 | 0.8 | 1 | 1 | 0.9 |

Bei keiner der Katzen wurden irgendwelche Nebenwirkungen festgestellt.

## Patentansprüche

1. Repellierende Zusammensetzung zur topischen Anwendung auf Tieren, bestehend aus 3 bis 12 Gew.-% einer Wirkstoffkomponente, 0 bis 5 Gew.-% Hilfsstoffen und dem Rest Isopropyllaurat und Isopropylmyristat in einem Verhältnis von Isopropyllaurat zu Isopropylmyristat von 70:30 bis 100:0, wobei die Wirkstoffkomponente aus einem oder mehreren natürlichen Repellents besteht, von denen mindestens 90 Gew.-% Margosa-Extrakt oder einer Mischung aus Margosa-Extrakt und Decansäure sind.

2. Zusammensetzung gemäss Anspruch 1, **dadurch gekennzeichnet, dass** die Spreitmittelkomponente mindestens zu 90 Gew.-% aus Isopropyllaurat besteht.

3. Zusammensetzung gemäss Anspruch 1 oder 2, **dadurch gekennzeichnet, dass** die Spreitmittelkomponente zu ca. 100 Gew.-% aus Isopropyllaurat besteht.

4. Zusammensetzung nach Anspruch 1, **dadurch gekennzeichnet, dass** sie aus 3 bis 12 Gew.-% Wirkstoffkomponente, 0 bis 1 Gew.-% Hilfsstoffen, und dem Rest Isopropyllaurat und Isopropylmyristat in einem Verhältnis von Isopropyllaurat zu Isopropylmyristat von 70:30 bis 100:0 besteht.

5. Zusammensetzung nach Anspruch 4, **dadurch gekennzeichnet, dass** das Verhältnis von Isopropyllaurat zu Isopropylmyristat von 80:20 bis 100:0 beträgt.

6. Zusammensetzung nach Anspruch 4, **dadurch gekennzeichnet, dass** das Verhältnis von Isopropyllaurat zu Isopropylmyristat von 90:10 bis 100:0 beträgt.

7. Zusammensetzung gemäss einem der vorangehenden Ansprüche, **dadurch gekennzeichnet, dass** in der Wirkstoffkomponente neben Margosa-Extrakt und Decansäure enthaltene natürliche Repellents ausgewählt sind aus der Gruppe bestehend aus etherischen Ölen und Mischungen von etherischen Ölen.

8. Zusammensetzung gemäss einem der vorangehenden Ansprüche, **dadurch gekennzeichnet, dass** die Wirkstoffkomponente ausgewählt ist aus der Gruppe bestehend aus Margosa-Extrakt, und einer Mischung aus Margosa-Extrakt und Decansäure im Verhältnis 30 : 70 bis 70 : 30.

9. Zusammensetzung gemäss Anspruch 8, **dadurch gekennzeichnet, dass** das Verhältnis Margosa-Extrakt zu Decansäure 40 : 60 bis 60 : 40, speziell bevorzugt ca. 50 : 50 beträgt.

10. Zusammensetzung gemäss einem der vorangehenden Ansprüche, **dadurch gekennzeichnet, dass** die Wirkstoffkomponente in Mengen von 4 bis 8 Gew.-%, insbesondere von 5 bis 8 Gew-% vorhanden ist.

11. Zusammensetzung gemäss einem der Ansprüche 1 bis 9, **dadurch gekennzeichnet, dass** die Wirkstoffkomponente in Mengen von 8 bis 12 Gew.-%, insbesondere von 9 bis 10 Gew-% vorhanden ist.

12. Zusammensetzung gemäss einem der vorangehenden Ansprüche, **dadurch gekennzeichnet, dass** die Tiere Haus- und/oder Heimtiere sind, insbesondere Hunde und/oder Katzen und/oder Pferde, speziell bevorzugt Hunde und/oder Katzen.

13. Zusammensetzung gemäss einem der vorangehenden Ansprüche, **dadurch gekennzeichnet, dass** sie eine Spot-on-Formulierung ist.

14. Zusammensetzung gemäss einem der Ansprüche 1 bis 12, **dadurch gekennzeichnet, dass** sie auf einem saugfähigen Material, insbesondere dem Teil eines Halsbands, aufgebracht vorliegt.

15. Verwendung einer Zusammensetzung gemäss einem der vorangehenden Ansprüche zum Repellieren von Ektoparasiten.

## Claims

1. Repellent composition for the topical use on animals, consisting of 3 to 12% by weight of an active substance component, 0 to 5% by weight of adjuvants and the remainder isopropyl laurate and isopropyl myristate in an isopropyl laurate to isopropyl myristate ratio of from 70:30 to 100:0, wherein the active substance component consists of one or more natural repellents, of which at least 90% by weight are Margosa extract or a mixture of Margosa extract and decanoic acid.

2. Composition according to Claim 1, **characterized in that** the spreading agent component consists to at least 90% by weight of isopropyl laurate.

3. Composition according to Claim 1 or 2, **characterized in that** the spreading agent component consists to approximately 100% by weight of isopropyl laurate.

4. Composition according to Claim 1, **characterized in that** it consists of 3 to 12% by weight of active substance component, 0 to 1% by weight of adjuvant and the remainder isopropyl laurate and isopropyl myristate in an isopropyl laurate to isopropyl myristate ratio of from 70:30 to 100:0.

5. Composition according to Claim 4, **characterized in that** the isopropyl laurate to isopropyl myristate ratio is from 80:20 to 100:0.

6. Composition according to Claim 4, **characterized in that** the isopropyl laurate to isopropyl myristate ratio is from 90:10 to 100:0.

7. Composition according to one of the preceding claims, **characterized in that** natural repellents present in the active substance component in addition to Margosa extract and decanoic acid are selected from the group consisting of essential oils and mixtures of essential oils.

8. Composition according to one of the preceding claims, **characterized in that** the active substance component is selected from the group consisting of Margosa extract and a mixture of Margosa extract and decanoic acid in the ratio 30:70 to 70:30.

9. Composition according to Claim 8, **characterized in that** the ratio of Margosa extract to decanoic acid is 40:60 to 60:40, specifically preferably approximately 50:50.

10. Composition according to one of the preceding claims, **characterized in that** the active substance component is present in amounts of from 4 to 8% by weight, in particular from 5 to 8% by weight.

11. Composition according to one of Claims 1 to 9, **characterized in that** the active substance component is present in amounts of from 8 to 12% by weight, in particular from 9 to 10% by weight.

12. Composition according to one of the preceding claims, **characterized in that** the animals are domestic animals and/or pets, in particular dogs and/or cats and/or horses, specifically preferably dogs and/or cats.

13. Composition according to one of the preceding claims, **characterized in that** it is a spot-on formulation.

14. Composition according to one of Claims 1 to 12, **characterized in that** it is present in a form where it has been applied to an absorbent material, in particular part of a collar.

15. Use of a composition according to one of the preceding claims for repelling ectoparasites.

## Revendications

1. Composition répulsive destinée à l'application topique sur des animaux, constituée de 3 à 12 % en poids d'un composant principe actif, 0 à 5 % en poids d'adjuvants et pour le reste de laurate d'isopropyle et myristate d'isopropyle en un rapport du laurate d'isopropyle au myristate d'isopropyle de 70:30 à 100:0, le composant principe actif consistant en un ou plusieurs répulsifs naturels, dont au moins 90 % en poids d'extrait de margosa ou d'un mélange d'extrait de margosa et d'acide décanoïque.

2. Composition selon la revendication 1, **caractérisée en ce que** le composant agent d'étalement consiste au moins à raison de 90 % en poids en laurate d'isopropyle.

3. Composition selon la revendication 1 ou 2, **caractérisée en ce que** le composant agent d'étalement consiste à raison d'environ 100 % en poids en laurate d'isopropyle.

4. Composition selon la revendication 1, **caractérisée en ce qu'**elle est constituée de 3 à 12 % en poids de composant principe actif, 0 à 1 % en poids d'adjuvants et pour le reste de laurate d'isopropyle et myristate d'isopropyle en un rapport du laurate d'isopropyle au myristate d'isopropyle de 70:30 à 100:0.

5. Composition selon la revendication 4, **caractérisée en ce que** le rapport du laurate d'isopropyle au myristate d'isopropyle vaut de 80:20 à 100:0.

6. Composition selon la revendication 4, **caractérisée en ce que** le rapport du laurate d'isopropyle au myristate d'isopropyle vaut de 90:10 à 100:0.

7. Composition selon l'une quelconque des revendications précédentes, **caractérisée en ce que** les répulsifs naturels, contenus en plus d'extrait de margosa et d'acide décanoïque dans le composant principe actif, sont choisis dans le groupe constitué par des huiles essentielles et des mélanges d'huiles essentielles.

8. Composition selon l'une quelconque des revendications précédentes, **caractérisée en ce que** le composant principe actif est choisi dans le groupe constitué par l'extrait de margosa et un mélange d'extrait de margosa et d'acide décanoïque en un rapport de 30 : 70 à 70 : 30.

9. Composition selon la revendication 8, **caractérisée en ce que** le rapport extrait de margosa à acide décanoïque vaut de 40 : 60 à 60 : 40, de façon particulièrement préférée environ 50 : 50.

10. Composition selon l'une quelconque des revendications précédentes, **caractérisée en ce que** le composant principe actif est présent en des quantités de 4 à 8 % en poids, en particulier de 5 à 8 % en poids.

11. Composition selon l'une quelconque des revendications précédentes, **caractérisée en ce que** le composant principe actif est présent en des quantités de 8 à 12 % en poids, en particulier de 9 à 10 % en poids.

12. Composition selon l'une quelconque des revendications précédentes, **caractérisée en ce que** les animaux sont des animaux domestiques et/ou de compagnie, en particulier des chiens et/ou des chats et/ou des chevaux, de façon particulièrement préférée des chiens et/ou des chats.

13. Composition selon l'une quelconque des revendications précédentes, **caractérisée en ce qu'**elle est une formulation « spot-on ».

14. Composition selon l'une quelconque des revendications 1 à 12, **caractérisée en ce qu'**elle se trouve appliquée sur un matériau absorbant, en particulier la partie d'un collier.

15. Utilisation d'une composition selon l'une quelconque des revendications précédentes, pour la répulsion d'ectoparasites.
